# FASCICULE DE BREVET EUROPEEN

(11) **EP 1 492 653 B1**
(45) Date de publication et mention de la délivrance du brevet: **31.05.2006**
(21) Numéro de dépôt: 03738219.9
(22) Date de dépôt: 09.04.2003
(51) Int. Cl.: B27K 1/00

(54) **PROCEDE DE TRAITEMENT DE MATIERES LIGNOCELLULOSIQUES, NOTAMMENT DU BOIS**
VERFAHREN ZUR BEHANDLUNG VON LIGNOCELLULOSISCHEN MATERIALIEN, INSBESONDERE FÜR HOLZ
METHOD FOR TREATING LIGNOCELLULOSIC MATERIALS, IN PARTICULAR WOOD

(30) Priorité: 10.04.2002 FR 0204448
(43) Date de publication de la demande: 05.01.2005
(60) Demande divisionnaire: 06100897.5
(73) Titulaire: Lapeyre, 93304 Aubervilliers (FR)
(72) Inventeur: MAGNE, Michel, F-51530 Moussy (FR); EL KASMI, Silham, F-51460 L Epine (FR); DUPIRE, Maxime, F-31400 Toulouse (FR); MORARD, Marie, F-31100 Toulouse (FR); VACA-GARCIA, Carlos, F-31500 Toulouse (FR); THIEBAUD-ROUX, Sophie, F-31240 L'Union (FR); PEYDECASTAING, Jérôme, F-31200 Toulouse (FR); BORREDON, Elisabeth, F-31170 Tournefeuille (FR); GASET, Antoine, F-31000 Toulouse (FR)
(74) Mandataire: Jamet, Vincent
(86) Numéro de dépôt international: PCT/FR2003/001110
(87) Numéro de publication internationale: WO 2003/084723

(56) Documents cités:
- DATABASE CA [en ligne] CHEMICAL ABSTRACTS SERVICE, COLUMBUS, OHIO, US; DAWSON, BERNARD S. W. ET AL: "Reactivity of radiata pine sapwood towards carboxylic acid anhydrides" retrieved from STN Database accession no. 130:268671 CA XP002222940 & HOLZFORSCHUNG (1999), 53(2), 195-198 , 1999,
- DATABASE CA [en ligne] CHEMICAL ABSTRACTS SERVICE, COLUMBUS, OHIO, US; VACA-GARCIA, C. ET AL: "Cellulose esterification with fatty acids and acetic anhydride in lithium chloride/N,N-dimethylacetamide medium" retrieved from STN Database accession no. 128:168867 CA XP002255518 & JOURNAL OF THE AMERICAN OIL CHEMISTS' SOCIETY (1998), 75(2), 315-319 , 1998,
- DATABASE CA [en ligne] CHEMICAL ABSTRACTS SERVICE, COLUMBUS, OHIO, US; SOC. ANON. DITE. PROGIL. PATENT NO. KIND DATE APPLICATION NO. DATE: "Coating composition containing phenolic resins" retrieved from STN Database accession no. 18:22009 CA XP002255519 & GB 215 722 1 (SOC. ANON. DITE. PROGIL. PATENT NO. KIND DATE APPLICATION NO. DATE) 11 décembre 1924 (1924-12-11)

## Description

La présente invention est relative à un procédé de traitement de matières lignocellulosiques, notamment du bois.

Elle vise plus particulièrement un procédé de protection du bois permettant de lui conférer un caractère hydrophobe, afin d'augmenter sa durabilité et sa stabilité dimensionnelle.

On sait qu'à l'état naturel le bois, ou plus précisément les fibres de bois qui sont en contact d'une atmosphère humide, ont tendance à se gorger d'eau. Cette absorption d'eau s'accompagne d'un gonflement.

Afin d'éliminer cette eau, on peut procéder à un séchage. Toutefois, bien que l'étape de séchage permette d'éliminer l'eau du bois, elle ne modifie en rien son caractère naturel hydrophile, si bien que la pièce de bois est de nouveau capable de réabsorber de l'eau éliminée lors du séchage lorsque cette pièce se trouve de nouveau dans un environnement humide.

Afin de diminuer, voire supprimer le caractère hydrophile du bois et lui conférer ainsi une stabilité dimensionnelle à long terme (classiquement une dizaine d'années), on a recherché des techniques de traitement.

Parmi celles-ci, on peut relever deux grandes familles qui se différencient en des procédés physiques de traitement thermique (généralement à des températures supérieures à 150°C) et des procédés de traitement physico-chimique, généralement à des températures inférieures à 120°C.

La présente invention s'intéresse aux procédés de traitements physicochimiques.

Parmi ces traitements physico-chimiques, on connaît notamment par plusieurs publications Ami et coll., (Ami, 1961); Matsuzaki et coll., des procédés permettant de synthétiser des esters mixtes à partir d'un milieu trifluoroacétique. Ces procédés ne sont pas viables industriellement en raison de l'utilisation de solvant et de catalyseur toxiques.

Des études complémentaires menées sur des sciures de bois ont démontré que l'estérification en présence d'une catalyse acide fort (se rajoutant à l'acidité intrinsèque du milieu) permettait de conférer à ces sciures un caractère hydrophobe. Ces études ont fait l'objet de la publication suivante Vaca-Garcia C ;, Borredon M.E, 1999, Solvent-free fatty acylation of cellulose and lignocellulosic wastes. Part 2 : reactions with fatty acids, Bioresource Technology, 70, 135-142.

Les inconvénients majeurs ce procédé en présence d'une catalyse acide résident dans la perte de masse de la sciure de bois, cette perte de masse résultant d'une dégradation des biopolymères constituant la sciure. On peut remarquer également un changement de couleur de la sciure après traitement.

Une technique similaire à la précédente n'est pas transposable sur une pièce de bois. En effet, on a constaté que les molécules d'hémicelluloses et la
cellulose sont hydrolysées partiellement, ce qui entraîne une diminution du poids moléculaire par la formation d'oligomères ainsi qu'une diminution des propriétés mécaniques, ainsi qu'une dégradation esthétique de la pièce de bois traitée.

La présente invention vise à pallier ces inconvénients en proposant un procédé qui confère au bois massif un caractère hydrophobe, tout en garantissant une stabilité dimensionnelle dans le temps, sans provoquer la création de gerces, de craquellements, de fendillements, ni changement de couleur.

La présente invention a ainsi pour objet un procédé de traitement chimique de matières lignocellulosiques, notamment d'au moins une pièce de bois, qui se caractérise en ce que l'on soumet lesdites matières à une imprégnation par un agent chimique comportant des chaînes hydrocarbonées, cet agent étant choisi parmi les anhydrides mixtes, sauf l'anhydride mixte d'acide acétique/benzoïque, ledit agent étant adapté pour assurer un greffage par liaison covalente d'une pluralité de chaînes hydrocarbonées sur lesdites matières.

Grâce à ces dispositions, on améliore la protection en surface et à coeur de la matière lignocellulosique, notamment du bois, par modification de ses fonctions hydroxyles.

Dans des modes de réalisation préférés de l'invention, on peut éventuellement avoir recours en outre à l'une et/ou à l'autre des dispositions suivantes :
- le greffage s'effectue par un processus d'estérification desdites matières lignocellulosiques à l'aide d'un agent chimique choisi parmi les anhydrides organiques,
- le traitement s'effectue à une température comprise entre la température ambiante et 150 °C, et de préférentiellement entre 100 et 140 °C,
- l'anhydride organique est un anhydride mixte,
- l'anhydride mixte comporte une première chaîne hydrocarbonée R et une seconde chaîne hydrocarbonée R₁,
- RCOOH represente un acide carboxylique de C2 à C4 et R₁COOH est un acide gras de C6 à C24 saturés ou insaturés,
- R₁COOH représente un acide carboxilique de C2 à C4 et RCOOH est un acide gras de C6 à C24 saturés ou insaturés,
- l'anhydride mixte est l'anhydride mixte d'acides acétique/octanoïque.
- l'imprégnation s'effectue en présence d'un catalyseur basique,
- l'imprégnation s'effectue en présence d'un catalyseur neutre,
- l'imprégnation s'effectue en présence d'un catalyseur acide faible,
- l'imprégnation s'effectue sans présence de catalyseur.
- l'imprégnation des matières lignocellulosiques est réalisée par un procédé de trempage,
- l'imprégnation des matières lignocellulosiques est réalisée par un procédé d'aspersion,
- l'imprégnation des matières lignocellulosiques est réalisée au sein d'un autoclave,
- le procédé de traitement est réalisé sur une pièce de bois, dont l'essence est choisie parmi notamment le chêne, le pin, le sapin, le curupixa, l'eucalyptus.

Une pièce de bois traité selon le procédé visé précédemment se caractérise en ce que les fibres ligno-cellulosiques sont homogène et présentent un aspect lissé,

Dans des modes de réalisation préférés de l'invention, on peut éventuellement avoir recours en outre à l'une et/ou à l'autre des dispositions suivantes:
- le taux d'absorption des fibres lignocellulosiques traitées est sensiblement voisin de 3.5 %,
- le taux de gonflement des fibres lignocellulosiques traitées est sensiblement voisin de 3.5 %.

D'autres caractéristiques et avantages de l'invention apparaitront au cours de la description suivante d'une de ses formes de réalisation, donnée à titre d'exemple non limitatif, en regard des dessins joints.

Sur les dessins :
- La figure 1 est une vue prise au microscope à balayage (MEB) d'un échantillon de bois non traité, il peut servir de référence.
- La figure 2 est une vue prise au microscope à balayage (MEB) d'un échantillon de bois ayant subi le procédé objet de l'invention, en présence d'un catalyseur acide fort.
- La figure 3 est une autre vue prise au microscope à balayage (MEB) d'un échantillon de bois ayant subi le procèdé objet de l'invention, en présence d'un catalyseur acide fort.

Selon un mode préféré de réalisation du procédé objet de l'invention, celui-ci consiste à imprégner des matières lignocellulosiques, telles que notamment au moins une pièce de bois par un agent chimique comportant des chaînes hydrocarbonées, ledit agent étant adapté pour assurer un greffage par liaison covalente d'une pluraritê de chaînes hydrocarbonées sur lesdites matières.

On entend par chaîne hydrocarboné toute chaîne hétéro aliphatique, hétéro aromatique, aliphatique, ou aromatique.

Cette imprégnation est réalisée à une température comprise entre la température ambiante et 150°C et préférentiellement entre 100 et 140°C.

Cet agent chimique est choisi parmi les anhydrides organiques, et préférentiellement parmi les anhydrides mixtes.

Préalablement à la phase d'imprégnation par l'agent chimique desdites matières lignocelllulosiques (par exemple au moins une pièce de bois), on procède à une étape de préparation de l'anhydride mixte.

Selon une première méthode : à partir d'un chlorure d'acide et d'un acide carboxylique selon la réaction suivante : 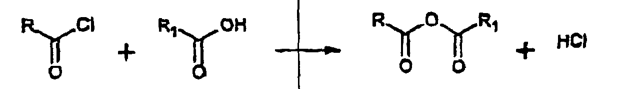 Selon une variante de la première méthode, consistant à échanger la position de R et de R₁

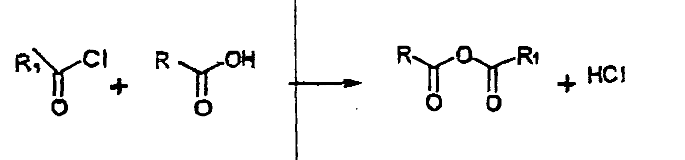

Selon une deuxième méthode: à partir d'un chlorure d'acide et d'un sel d'acide carboxylique selon la réaction suivante :

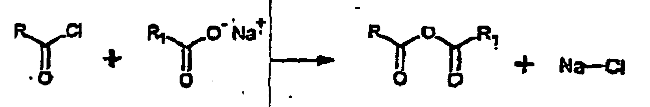

Selon une troisième méthode : à partir d'un anhydride d'acide carboxylique linéaire et d'un acide gras, selon la réaction suivante.

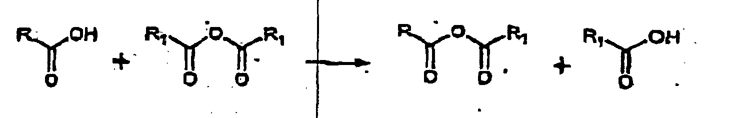

Les radicaux R, R₁ sont des chaînes aliphatiques de longueurs différentes. A titre d'exemple non limitatif, on pose que R est de longueur plus petite que R₁.

RCOOH représente par un exemple un acide carboxylique de C2 à C4 (acétique, propionique ou butyrique tandis que R₁ COOH est un acide gras de C6 à C24 saturés ou insaturés (hexylique, octanoïque ou oléique par exemple).

Les anhydrides mixtes peuvent être utilisés purs ou en mélange, et dans ce cas provenir être issus d'un mélange de différents acides carboxyliques, à partir desquels on réalise la synthèse de l'anhydride mixte recherché.

A partir de l'anhydride mixte obtenu par l'une au moins des méthodes mentionnées précédemment, on procède alors à l'imprégnation d'une pièce de bois, de manière à greffer l'anhydride mixte (par exemple de l'anhydride acétique/octanoïque) sur ladite pièce de bois, ce greffage consistant en une estérification du bois selon la réaction suivante :

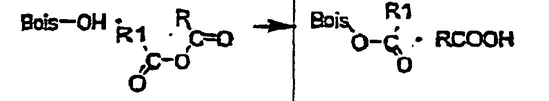

Ou inversement au niveau du rôle entre R et R1

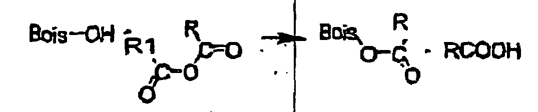

D'autres méthodes d'estérification peuvent être également utilisées selon les réactions envisagées ci-après :

A partir d'un chlorure d'acide, cette réaction est rapide mais le dégagement de HCl constitue un inconvénient majeur.

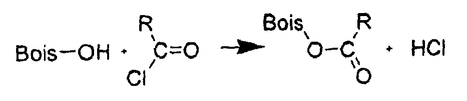

A titre d'exemple, le chlorure d'acide est choisi parmi le chlorure d'octanoyle, le chlorure d'acétoyle.

A partir d'un cétène, les réactifs sont cependant chers, ce qui limite l'intérêt industriel.

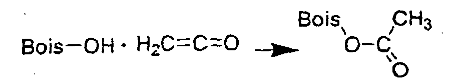

A titre d'exemple, cette réaction peut être associée avec par exemple le chlorure d'octanoyle.

A partir d'acides carboxyliques, cette réaction présente néanmoins une faible réactivité et nécessite l'utilisation de co-réactifs : Pyridine, DCC, TsCl, TFAA (DCC : N,N-dicyclohexylcarbodiimide ; TsCl : Chlorure de p-toluènesulfonyle ; TFAA : Anhydride trifluoroacétique)

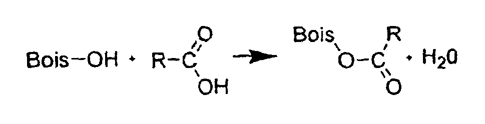

A titre d'exemples, les acides carboxyliques utilisés sont choisis parmi l'acide acétique, l'acide octanoïque.

A partir d'esters d'acides carboxyliques (par exemple de l'octanoate de méthyle, de l'acétate de méthyle), on peut remarquer cependant que si R consiste en du CH3, il se produit un dégagement de méthanol (toxique).

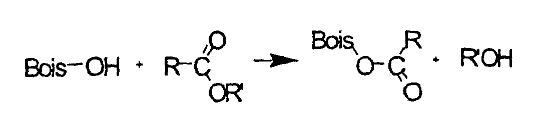

Les esters mixtes de bois peuvent être obtenus soit
- en une seule étape par un mélange des réactifs choisis parmi ceux présentés précédemment
- ou bien en 2 étapes et ce,
   o soit en utilisant deux fois le même type de réaction
   o soit avec deux réactions de deux familles différentes.

En outre, selon une caractéristique de l'invention, ces réactions d'estérification peuvent avoir lieu sans présence de catalyseur, ou avec présence de catalyseur basique ou neutre (tel que par exemple du carbonate de calcium, carbonate de sodium, carbonate de potassium, sel d'acide gras...) ou bien avec un catalyseur acide faible ou encore avec un catalyseur acide fort dont les effets néfastes sur le bois sont minimisés par l'utilisation de concentrations très diluées.

On donnera ci-après un exemple de mise en oeuvre du procédé selon l'invention :
Exemple 1 : Une mole d'anhydride acétique a été ajoutée à une mole d'acide octanoïque. Le mélange a été chauffé sous agitation à 140°C pendant 30 minutes. Une pièce de bois de dimensions 10*10*10 cm a été ensuite plongée dans le mélange réactionnel et le tout a été chauffé à 140°C pendant 1 heure. La pièce de bois est ensuite égouttée et mise à sécher dans un four ventilé.
Exemple 2 : Une mole d'anhydride acétique a été ajoutée à une mole d'acide octanoïque. Le mélange a agité à température ambiante pendant 60 minutes. Une pièce de bois de dimensions 10*10*10 cm a été ensuite plongée dans le mélange réactionnel pendant 5 minutes puis égoutée. La pièce de bois a été introduite dans un four à 120°C pendant 1 heure.

Un avantage majeur de la présente invention consiste dans le recours en un anhydride mixte d'origine végétale, non toxique, par opposition à des composés d'origine pétrochimique.

Ce choix particulier favorise la mise en oeuvre industrielle de l'invention, car il simplifie les traitements qui visent à préserver l'environnement.

Quel que soit le procédé de traitement utilisé, il convient de pouvoir retrouver a posteriori la signature de ce traitement sur la matière lignocellulosique (dans notre cas d'espèce une pièce de bois).

Différentes méthodes sont envisagées permettant de caractériser le traitement qu'a subi la matière lignocellulosique, à savoir la détermination de la présence de chaînes hydrocarbonées différentes liées par des fonctions esters ainsi que de la présence ou non d'un catalyseur (et son type).

Une méthode permettant de déterminer la présence de chaînes hydrocarbonées consiste à traiter un échantillon provenant de la pièce de bois par une solution de NaOH afin d'hydrolyser les fonctions esters et transformet les chaînes hydrocarbonées en acide carboxylique. Ces derniers sont ensuite identifiés par des méthodes classiques chromatographiques telles que HPLC, GC, etc...

Un exemple de ces méthodes consister, à partir d'une pièce de bois ou d'un matériau lignocellulosique dont les fonctions hydroxyles ont été acylées par au moins deux agents hydrocarbonés différents, à donner lieu à des mélanges d'esters, par exemple des acétates et des octanoates de matière lignocellulosique.

Ce mélange d'esters peut être caractérisé de la façon suivante : un échantillon de bois ou de matière lignocellufosique traité par le procédé revendiqué est broyé jusqu'à une granulométrie d'au moins 80 mesh puis introduit dans une fiole contenant une solution aqueuse d'éthanol (70%). Après agitation pendant au moins une heure, une quantité suffisante d'une solution aqueuse de NaOH (0,5 M) est ajoutée et l'agitation est poursuivie pendant 72 h pour effectuer une saponification totale des fonctions esters. Après filtration et séparation du résidu solide, le liquide est acidifié à pH 3 avec une solution aqueuse de HCl (1 M) afin de convertir les composés hydrocarbonés en acides carboxyliques correspondants. Le liquide peut ensuite être analysé par chromatographie en phase gazeuse (CPG) ou bien par chromatographie liquide haute performance (HPLC) afin de séparer et identifier les différents acides carboxyliques correspondant aux fonctions esters présents dans le bois ou matériau lignocellulosique traité.

On donnera ci-après des méthodes permettant d'identifier le type de catalyseur.

Ainsi une première méthode consiste à procéder à une détermination de la quantité d'extractibles. Cette méthode permet d'observer l'influence des divers traitements sur les extractibles du bois (initialement présents, ou issus de la dégradation du bois). On fait subir au bois traité puis micronisé des extractions avec plusieurs solvants, de polarités différentes : l'eau, l'éthanol, l'acétone, et le cyclohexane. Les extractions sont réalisées à l'aide d'appareil de Soxhlet

Dans le tableau ci-après sont regroupées les quantités d'extractibles des échantillons de bois traités, après extraction au Soxhlet avec divers solvants.

| | PERTE de MASSE (%) APRES EXTRACTION | | | |
|---|---|---|---|---|
| | Eau | Ethanol | Acétone | Cyclohexane |
| Sans catalyse | 14.8 | 11.9 | 12.2 | 6.3 |
| Catalyse basique | 17.1 | 16.2 | 10.6 | 1.8 |
| Catalyse Acide fort | 25.3 | 21.7 | 19.0 | 4.8 |

Comme on peut le voir, quel que soit le solvant d'extraction, ces résultats confirment les impressions visuelles : le traitement en catalyse acide fort (H₂SO₄ 0.3% molaire) qui est le plus dégradant et qui conduit à la formation de la plus grande quantité de composés extractibles en fin de réaction. Pour des quantités d'acide fort importantes (0.3% molaire), la pièce de bois noircie et a tendance à se désagréger et à présenter des défauts d'aspect.

A l'échelle microscopique, la paroi cellulaire des fibres est abîmée du fait de la catalyse acide.

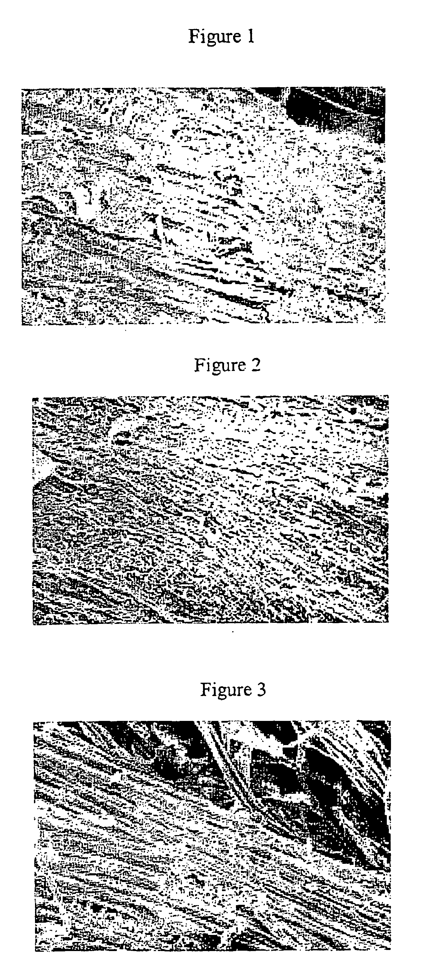

Ainsi, par rapport à la figure 1, et d'un point de vue qualitatif, on peut constater au niveau de la figure 2, que la surface du bois semble avoir été lissée par le traitement, cette surface du bois est homogène. Les fibres du bois (ligno-cellulosiques) visibles au microscope semblent intactes comparées à celles de la Figure 1. Le produit semble d'une part avoir une sorte d'action de décapage de la surface mais également permet une homogénéisation de la surface grâce au greffage. En effet, les chaînes greffées sont susceptibles de protéger les fibres ce qui les rend indiscernables au microscope.

De même au niveau de la figure 3, les fibres ligno-cellulosiques semblent être à nu. La présence de produit est beaucoup moins nette que précédemment (figure 2) ceci est logique car la photographie présente l'intérieur d'un bloc traité par le procédé d'invention. Le déchiquetage est dû soit au traitement, soit, probablement à l'arrachement des fibres lors de la découpe.

D'un point de vue quantitatif, on donne ci après un tableau qui exprime les valeurs d'absorption et de gonflement pour des fibres ligno-cellulosiques traitées et non traitées.

| | Fibres non traitées | Fibres traitées |
|---|---|---|
| Absorption en % | 16 | 3.5 |
| Gonflement en % | 6.5 | 3.5 |

Une seconde méthode consiste à une analyse des constituants du bois. Suivant le type de milieu dans lequel le bois est traité, les biopolymères du bois ne subissent pas tous les mêmes dégradations. La composition du bois traité est donc susceptible de varier en fonction du traitement. Cette méthode est dite ADF-NDF, et elle permet de connaître les proportions de cellulose C, d'hémicelluloses H, de lignines L, de matière minérale MM

Dans le tableau ci-après sont regroupées les données relatives à l'analyse de la composition du bois de chêne traité avec l'anhydride mixte acétiqueoctanoïque avec différents types de catalyseurs. Les échantillons estérifiés ont été saponifiés selon le protocole d'analyse des esters mixtes de bois puis lavés par extraction à l'eau à l'aide d'un appareil de Soxhlet avant d'être analysés par la technique ADF-NDF. Cette technique se trouve décrite dans la référence (Acid Detergent Fiber, Neutral Detergent Fiber) VAN SOEST P.J. and WINE R.H. Determination of lignin and cellulose in acid-detergent fiber with permanganate. J. *Ass. Offic. Anal. Chem.* 51(4), 780-785 (1968).

| Nature du traitement | Catalyseur | Extractibles (%) | Cellulose (%) | Hémicelluloses (%) | Lignine (%) | Divers (%) | Cendres (%) |
|---|---|---|---|---|---|---|---|
| Bois non traité | - | 5.0 | 50.9 | 17.6 | 20.5 | 5.4 | 0.6 |
| Catalyse acide fort | H₂SO₄ 0.3 %mol | 22.4 | 49.7 | 14.7 | 8.5 | 4.4 | 0.3 |
| Catalyse Basique | Na₂CO₃ 0.3 %mol | 16.9 | 40.6 | 16.4 | 20.1 | 5.7 | 0.3 |
| Sans catalyse | - | 12.5 | 41.4 | 17.5 | 17.1 | 10.8 | 0.7 |

Cette analyse permet donc de distinguer un traitement avec catalyse acide fort des traitements revendiqués. En effet, on remarque une diminution importante et significative de la quantité de lignine et des hémicelluloses. De plus, la quantité d'extractibles au soxhlet à l'eau est la plus importante.

## Revendications

1. Procédé de traitement chimique de matières lignocellulosiques, notamment d'au moins une pièce de bois, ***caractérisé en ce que*** l'on soumet lesdites matières à une imprégnation par un agent chimique comportant des chaînes hydrocarbonées, cet agent étant choisi parmi les anhydrides mixtes, sauf l'anhydride mixte d'acide acétique/benzoïque, ledit agent étant adapté pour assurer un greffage par liaison covalente d'une pluralité de chaînes hydrocarbonées sur lesdites matières.

2. Procédé selon la revendication 1, ***caractérisé en ce que*** le greffage s'effectue par un processus d'estérification desdites matières lignocellulosiques à l'aide d'un agent chimique choisi parmi les anhydrides organiques.

3. Procédé selon les revendications 1 ou 2, ***caractérisé en ce que*** le traitement s'effectue à une température comprise entre la température ambiante et 150 °C, et de préférentiellement entre 100 et 140 °C.

4. Procédé selon la revendication 1, ***caractérisé en ce que*** l'anhydride mixte comporte une première chaîne hydrocarbonée R et une seconde chaîne hydrocarbonée R₁.

5. Procédé selon la revendication 4, ***caratérisé en ce que*** RCOOH représente un acide carboxylique de C2 à C4 et R₁COOH est un acide gras de C6 à C24 saturés ou insaturés.

6. Procédé selon la revendication 4, ***caractérisé en ce que*** R₁COOH représente un acide carboxylique de C2 à C4 et RCOOH est un acide gras de C6 à C24 saturés ou insaturés.

7. Procédé selon la revendication 1, ***caractérisé en ce que*** l'anhydride mixte est l'anhydride mixte d'acides acétique/octanoïque.

8. Procédé selon l'une des revendications 1 à 7, ***caractérisé en* ce *que*** l'imprégnation s'effectue en présence d'un catalyseur basique.

9. Procédé selon l'une des revendications 1 à 7, ***caractérisé en ce que*** l'imprégnation s'effectue en présence d'un catalyseur neutre.

10. Procédé selon l'une des revendications 1 à 7, ***caractérisé en ce que*** l'imprégnation s'effectue en présence d'un catalyseur acide faible.

11. Procédé selon l'une des revendications 1 à 7, ***caractérisé en ce que*** l'imprégnation s'effectue sans présence de catalyseur.

12. Procédé selon l'une des revendications 1 à 7, ***caractérisé en ce que*** l"imprégnation des matières lignocellulosiques est réalisée par un procédé de trempage.

13. Procédé selon l'une des revendications 1 à 7, ***caractérisé en ce que*** l'imprégnation des matières lignocellulosiques est réalisée par un procédé d'aspersion.

14. Procédé selon l'une des revendications 1 à 7, ***caractérisé en ce que*** l'imprégnation des matières ligaocellulosiques est réalisée au sein d'un autoclave.

15. Procédé selon l'une des revendications 1 à 14, ***caractérisé en ce qu*'**il est mis en oeuvre sur une pièce de bois, dont l'essence est choisie parmi notamment le chêne, le pin, le sapin, le curupixa, l'eucalyptus,

## Claims

1. A process for the chemical treatment of lignocellulose materials, in particular of at least one piece of wood, ***characterized in that*** said materials are subjected to impregnation by a chemical agent comprising hydrocarbonaceous chains, this agent being chosen from mixed anhydrides, except for the mixed anhydride of acetic/benzoic acid, said agent being suitable for providing covalent grafting of a plurality of hydrocarbonaceous chains to said materials.

2. The process as claimed in claim 1, ***characterized in that*** the grafting is carried out by a process for the esterification of said lignocellulose materials using a chemical agent chosen from organic anhydrides.

3. The process as claimed in claims 1 or 2, ***characterized in that*** the treatment is carried out at a temperature between ambient temperature and 150°C and preferably between 100 and 140°C.

4. The process as claimed in claim 1, ***caracterized in** that* the mixed anhydride comprises a first hydrocarbonaceous chain R and a second hydrocarbonaceous chain R₁.

5. The process as claimed in claim 4, ***characterized in that*** RCOOH represents a C₂ to C₄ carboxylic acid and R₁COOH is a C₆ to C₂₄ fatty acid, these acids being saturated or unsaturated.

6. The process as claimed in claim 4, ***characterized in that*** R₁COOH represents a C₂ to C₄ carboxylic acid-and RCOOH is a C₆ to C₂₄ fatty acid, these acids being saturated or unsaturated.

7. The process as claimed in claim 1, ***characterized in that*** the mixed anhydride is the mixed anhydride of acetic/octanoic acids.

8. The process as claimed in one of claims 1 to 7, ***characterized in that*** the impregnation is carried out in the presence of a basic catalyst.

9. The process as claimed in one of claims 1 to 7, ***characterized in that*** the impregnation is carried out in the presence of a neutral catalyst.

10. The process as claimed in one of claims 1 to 7. ***characterized in that*** the impregnation is carried out in the presence of a weak acid catalyst.

11. The process as claimed in one of claims 1 to 7. ***characterized in that*** the impregnation is carried out in the presence of a catalyst.

12. The process as claimed in one of claims 1 to 7, ***characterized in that*** the impregnation of the lignocellulose materials is carried out by a dipping process.

13. The process as claimed in one of claims 1 to 7, ***characterized in that*** the impregnation of the lignocellulose materials is carried out by a spraying process.

14. The process as claimed in one of claims 1 to 7, ***characterized in that*** the impregnation of the lignocellulose materials is carried out in an autoclave.

15. The process as claimed in one of claims 1 to 14, ***characterized in that*** it is carried out on a piece of wood, the species of which is chosen from In particular oak, pine, fir, curupixa or eucalyptus.

## Patentansprüche

1. Verfahren zum chemischen Behandeln von Holzzellulose-Materialien, insbesondere mindestens eines Stückes Holz, **dadurch gekennzeichnet, dass** die besagten Materialien einer Tränkung durch einen chemischen, Kohlenwasserstoffketten enthaltenden Wirkstoff unterzogen werden, wobei dieser Wirkstoff aus Mischanhydriden, außer dem Mischanhydrid aus Essig-/Benzoesäure, ausgewählt wird, wobei der besagte Wirkstoff dazu geeignet ist, auf diese Materialien eine Mehrzahl von Kohlenwasserstoffketten durch Kovalenzbindung aufzupfropfen.

2. Verfahren nach Anspruch 1, **dadurch gekennzeichnet, dass** die Pfropfung durch einen Prozess der Veresterung der besagten Holzzellulose-Materialien mittels eines chemischen Wirkstoffs abläuft, der aus organischen Anhydriden ausgewählt ist.

3. Verfahren nach Anspruch 1 oder 2, **dadurch gekennzeichnet, dass** die Behandlung bei einer zwischen der Umgebungstemperatur und 150 °C liegenden Temperatur abläuft, und vorzugsweise zwischen 100 und 140 °C.

4. Verfahren nach Anspruch 1, **dadurch gekennzeichnet, dass** das Mischanhydrid eine erste Kohlenwasserstoffkette R und eine zweite Kohlenwasserstoffkette R1 enthält.

5. Verfahren nach Anspruch 4, **dadurch gekennzeichnet, dass** RCOOH eine Karboxylsäure von C2 bis C4 repräsentiert, und dass R₁COOH eine gesättigte oder ungesättigte Fettsäure von C6 bis C24 ist.

6. Verfahren nach Anspruch 4, **dadurch gekennzeichnet, dass** R₁COOH eine Karboxylsäure von C2 bis C4 repräsentiert, und dass RCOOH eine gesättigte oder ungesättigte Fettsäure von C6 bis C24 ist.

7. Verfahren nach Anspruch 1, **dadurch gekennzeichnet, dass** das Mischanhydrid das Mischanhydrid von Essig- und Oktansäure ist.

8. Verfahren nach einem der Ansprüche 1 bis 7, **dadurch gekennzeichnet, dass** die Tränkung in Anwesenheit eines basischen Katalysators ausgeführt wird.

9. Verfahren nach einem der Ansprüche 1 bis 7, **dadurch gekennzeichnet, dass** die Tränkung in Anwesenheit eines neutralen Katalysators ausgeführt wird.

10. Verfahren nach einem der Ansprüche 1 bis 7, **dadurch gekennzeichnet, dass** die Tränkung in Anwesenheit eines schwach sauren Katalysators ausgeführt wird.

11. Verfahren nach einem der Ansprüche 1 bis 7, **dadurch gekennzeichnet, dass** die Tränkung ohne Anwesenheit eines Katalysators ausgeführt wird.

12. Verfahren nach einem der Ansprüche 1 bis 7, **dadurch gekennzeichnet, dass** die Tränkung der Holzzellulose-Materialien durch ein Tauchverfahren ausgeführt wird.

13. Verfahren nach einem der Ansprüche 1 bis 7, **dadurch gekennzeichnet, dass** die Tränkung der Holzzellulose-Materialien durch ein Sprühverfahren ausgeführt wird.

14. Verfahren nach einem der Ansprüche 1 bis 7, **dadurch gekennzeichnet, dass** die Tränkung der Holzzellulose-Materialien in einem Autoklaven ausgeführt wird.

15. Verfahren nach einem der Ansprüche 1 bis 14, **dadurch gekennzeichnet, dass** es mit einem Stück Holz ausgeführt wird, dessen Art insbesondere aus Eiche, Kiefer, Tanne, Curupixa oder Eukalyptus ausgewählt ist.
